# EUROPEAN PATENT APPLICATION

(11) **EP 4 431 012 A1**
(43) Date of publication of application: **18.09.2024**
(21) Application number: 22909860.3
(22) Date of filing: 15.12.2022
(51) Int. Cl.: A61B 5/0225, A61B 5/00

(54) **BLOOD PRESSURE MEASUREMENT METHOD, AND ELECTRONIC DEVICE AND MEDIUM THEREOF**

(30) Priority: 21.12.2021 CN 202111569275; 12.04.2022 CN 202210383491
(71) Applicant: Huawei Technologies Co., Ltd., Shenzhen, Guangdong 518129 (CN)
(72) Inventor: JIA, Zheng, Shenzhen, Guangdong 518129 (CN); LI, Hongbao, Shenzhen, Guangdong 518129 (CN); ZENG, Yan, Shenzhen, Guangdong 518129 (CN); ZHOU, Jie, Shenzhen, Guangdong 518129 (CN); PENG, Jiahui, Shenzhen, Guangdong 518129 (CN); YAN, Jiabing, Shenzhen, Guangdong 518129 (CN)
(74) Representative: Maiwald GmbH
(86) International application number: PCT/CN2022/139279
(87) International publication number: WO 2023/116546

(57) **Abstract**

A blood pressure measurement method, an electronic device, and a medium are provided. In the blood pressure measurement method, whether to perform blood pressure measurement on a user, and a specific blood pressure measurement mode used to perform blood pressure measurement on the user are determined based on a degree of impact on a sleep status of the user. Measurement modes are classified into a first measurement mode and a second measurement mode. In the first measurement mode, the user needs to be prompted to adjust a blood pressure measurement posture to meet a blood pressure measurement requirement. After the user confirms that the posture is adjusted, blood pressure measurement is performed on the user. In the second measurement mode, blood pressure measurement can be performed on the user without prompting the user to adjust a blood pressure measurement posture, and finally, a blood pressure measurement value of the user is compensated based on an error between a blood pressure measurement value of the user with the posture and a blood pressure measurement value of the user with a posture used for blood pressure measurement. In this manner, impact of blood pressure measurement pressurization on the sleep status of the user can be reduced.

## Description

This application claims priorities to Chinese Patent Application No. 202111569275.3, filed with the China National Intellectual Property Administration on December 21, 2021 and entitled "DYNAMIC BLOOD PRESSURE MONITORING DEVICE AND METHOD", and to Chinese Patent Application No. 202210383491.7, filed with the China National Intellectual Property Administration on April 12, 2022 and entitled "BLOOD PRESSURE MEASUREMENT METHOD, ELECTRONIC DEVICE, AND MEDIUM", both of which are incorporated herein by reference in their entireties.

### TECHNICAL FIELD

This application relates to the field of physiological and health technologies, and in particular, to a blood pressure measurement method, an electronic device, and a medium.

### BACKGROUND

A wearable device, for example, a smartwatch, may have both functions of monitoring a sleep status of a user and measuring blood pressure of the user.

When the wearable device, for example, the smartwatch (referred to as a "watch" below) is used to perform dynamic blood pressure monitoring, there are usually two user postures: one is a sitting posture, and the other is a lying posture. The sitting posture means that the user sits on a chair, has a back support, has two feet on the ground, and lifts an arm to make the watch on a wrist level with the heart, and then a conventional oscillometric method is used to calculate blood pressure based on a pulse wave. The lying posture means that the user lies flat on a bed or lies on a side of the bed. In this case, there may be a height difference between the watch on the wrist and the heart. A gyroscope needs to be used to determine the lying posture, and then a blood pressure measurement deviation caused by the height difference is compensated for, that is, a corrected oscillometric method.

A specific manner of performing dynamic blood pressure monitoring is usually that the user sets blood pressure measurement start time and interval duration, and then blood pressure measurement is performed on the user at an interval of a period of time. However, when the user is in a sleep state, dynamic blood pressure monitoring is performed, and the wearable device may wake up the user in a process of pressurizing the wrist of the user, affecting sleep of the user.

### SUMMARY

To resolve the foregoing problem, this application provides a blood pressure measurement method, an electronic device, and a medium. The following describes this in detail.

According to a first aspect, an embodiment of this application provides a blood pressure measurement method, applied to a wearable device, where the wearable device includes a photoplethysmography PPG sensor, a micropump, and an airbag, and the method includes: determining a heart rate of a user based on the PPG sensor; if the heart rate of the user is greater than a first heart rate threshold, displaying a first user interface, where the first user interface is used to prompt the user to measure blood pressure; in response to a detected first operation performed on the first user interface, controlling the micropump to perform a first measurement operation on the airbag, where the first measurement operation is used to measure the blood pressure; if the heart rate of the user is less than a second heart rate threshold, determining whether a current moment is in a first time range, where the second heart rate threshold is less than or equal to the first heart rate threshold; and if the current moment is in the first time range, controlling the micropump to perform a second measurement operation on the airbag, where the second measurement operation is used to measure the blood pressure, where a same pressurization mode is used for the first measurement operation and the second measurement operation.

The heart rate of the user is used to determine a status of the user. For example, when the heart rate of the user is greater than the first heart rate threshold, it is determined that the user is in a waking state. In this case, corresponding prompt information may be displayed in the first user interface to prompt the user to adjust a posture to meet a blood pressure measurement requirement. After the user adjusts the posture, the user may confirm, by tapping the first operation, that the posture adjustment is completed, so that the wearable device can perform blood pressure measurement. Specifically, a blood pressure measurement process includes: The wearable device controls the micropump to perform the first measurement operation on the airbag, where the first measurement operation is used to control the micropump to perform inflating and deflating on the airbag. This blood pressure measurement mode is also referred to as the first measurement mode.

When the heart rate of the user is less than the second heart rate threshold, it is determined that the user is in a sleep state, and when the current moment is in the first time range, the user may not be prompted, and then the micropump is controlled to perform the second measurement operation on the airbag, where the second measurement operation is used to control the micropump to perform inflating and deflating on the airbag. This blood pressure measurement mode is also referred to as the second measurement mode.

In addition, the pressurization mode of the first measurement operation is the same as that of the second measurement operation. That is, in a pressurization process related to the first measurement operation and the second measurement operation, an upper limit value of pressure applied to a wrist of the user is related only to a blood pressure value of the user, and is unrelated to the status of the user.

The first time range is a night range. Alternatively, the first time range may be determined based on statistics collection on a work and rest habit of the user, or may be determined based on time range information input by the user in a related operation setting interface. Details are described in the following specific embodiment part.

According to this method, blood pressure measurement may be performed on the user in an appropriate measurement mode based on the status of the user, to reduce impact on the sleep status of the user.

In addition, different from an existing solution, when the user is in the sleep state, a manner of reducing the upper limit value of the pressure applied to the wrist of the user to perform blood pressure measurement on the user reduces accuracy of a blood pressure measurement result. In the blood pressure measurement method in this application, a pressurization process is the same in each measurement mode, that is, the upper limit value of the pressure applied to the wrist of the user is related only to the blood pressure value of the user, and is unrelated to the status of the user. Therefore, the upper limit value of the pressure applied to the wrist of the user does not decrease even if the user is in the sleep state. In this way, the blood pressure measurement method in this application also ensures accuracy of the blood pressure measurement result.

With reference to the first aspect, in a possible implementation of the first aspect, the wearable device further includes a gyroscope sensor and a pressure sensor, the pressure sensor is configured to detect pressure of the airbag, and the method further includes: in a process of the first measurement operation, determining a first signal based on the gyroscope sensor, and determining a second signal based on the pressure sensor; and if the first signal meets a first condition, displaying a second user interface after the first measurement operation, where the second user interface includes a first blood pressure value, and the first blood pressure value is determined based on the second signal; or if the first signal does not meet the first condition, displaying a third user interface, where the third user interface is used to inform the user that measurement is ineffective.

In other words, in the process of performing blood pressure measurement on the user in the first measurement mode, whether current blood pressure measurement is effective needs to be determined with reference to the first signal determined by the gyroscope sensor. If the first signal meets the first condition, it indicates that the current blood pressure measurement is effective, and a result of the current blood pressure measurement is displayed in the second user interface. If the first signal does not meet the first condition, it indicates that the current blood pressure measurement is ineffective, and the third user interface is used to inform the user that the current measurement is ineffective.

With reference to the first aspect, in a possible implementation of the first aspect, the first signal includes a first included angle that is between the wearable device and a horizontal plane and that is detected by the gyroscope sensor, and the first condition includes that the first included angle is in a first preset angle range. In other words, whether the current measurement is effective is determined by detecting the first included angle between the wearable device and the horizontal plane. When the first included angle is in the first preset angle range, it is determined that the current measurement is effective. When the first included angle is not in the first preset angle range, it is determined that the current measurement is ineffective. The first preset angle range is an empirical value or an experimental value. For example, the first preset angle range may be greater than 60° and less than 90°.

With reference to the first aspect, in a possible implementation of the first aspect, the third user interface is displayed after the first measurement operation ends. In other words, the user may be informed that the measurement is ineffective after the current measurement ends.

With reference to the first aspect, in a possible implementation of the first aspect, the wearable device further includes the gyroscope and the pressure sensor, the pressure sensor is configured to detect the pressure of the airbag, and the method further includes: in a process of the second measurement operation, determining a third signal based on the gyroscope sensor, and determining a fourth signal based on the pressure sensor; and determining a second blood pressure value based on the third signal and the fourth signal after the second measurement operation.

With reference to the first aspect, in a possible implementation of the first aspect, the third signal includes a second included angle that is between the wearable device and a horizontal plane and that is detected by the gyroscope sensor.

With reference to the first aspect, in a possible implementation of the first aspect, the determining a second blood pressure value based on the third signal and the fourth signal includes: determining a third blood pressure value based on the third signal, and determining a fourth blood pressure value based on the fourth signal; and determining the second blood pressure value based on the third blood pressure value and the fourth blood pressure value.

With reference to the first aspect, in a possible implementation of the first aspect, the method further includes: if the heart rate of the user is less than the second heart rate threshold, the current moment is in a second time range, and duration in which the heart rate of the user is less than the second heart rate threshold is greater than first preset duration, controlling the micropump to perform the second measurement operation on the airbag, where the second measurement operation is used to measure the blood pressure. In other words, when the user is in the sleep state and the current moment is in a day, there is a need to further determine whether duration in which the user is in the sleep state exceeds the first preset duration, to determine whether the user enters a deep sleep state. If the user enters the deep sleep state, the blood pressure of the user is measured in the second measurement mode. The first preset duration is an empirical value or an experimental value. For example, a value of the first preset duration may be 30 minutes.

With reference to the first aspect, in a possible implementation of the first aspect, the method further includes: if the heart rate of the user is greater than the first heart rate threshold, the current moment is in the first time range, and the current moment is in a first preset time period, displaying the first user interface, where the first user interface is used to prompt the user to measure the blood pressure; and in response to the detected first operation performed on the first user interface, controlling the micropump to perform the first measurement operation on the airbag. The first preset time period may be determined based on the statistics collection on the work and rest habit of the user. For example, if the user is often in a working state from 10:00 to 12:00 at night, the first preset time period is a working time period. In other words, when the user is in the waking state, the current moment is at night, and the current moment is also in the working time period, blood pressure measurement is performed on the user in the first blood pressure measurement mode.

With reference to the first aspect, in a possible implementation of the first aspect, the method further includes: if the heart rate of the user is greater than the first heart rate threshold, the current moment is in the second time range, and the current moment is in a second preset time period, skipping performing blood pressure measurement on the user. The second preset time period may be determined based on the statistics collection on the work and rest habit of the user. For example, if the user is often in an insomnia state from 10:00 to 12:00 at night, the first preset time period is an insomnia time period. In other words, when the user is in the waking state, the current moment is at night, and the current moment is also in the insomnia time period, to avoid affecting subsequent sleep of the user, blood pressure measurement is not performed on the user.

With reference to the first aspect, in a possible implementation of the first aspect, the method further includes: if the heart rate of the user is greater than the first heart rate threshold, the current moment is in the first time range, and duration in which the heart rate of the user is greater than the first heart rate threshold is less than second preset duration, skipping performing blood pressure measurement on the user. In other words, when the user is in the waking state, the current moment is at night, and the duration in which the heart rate of the user is greater than the first heart rate threshold is less than the second preset duration, it indicates that the user is in a get-up state at night or a short-time waking state at night. To avoid affecting subsequent sleep of the user, blood pressure measurement is not performed on the user. The second preset duration is an empirical value or an experimental value. For example, a value of the second preset duration may be 10 minutes.

With reference to the first aspect, in a possible implementation of the first aspect, the method further includes: if the heart rate of the user is greater than a third threshold and less than a fourth threshold, skipping performing blood pressure measurement on the user, where the third heart rate threshold is greater than or equal to the second heart rate threshold, and the fourth heart rate threshold is less than or equal to the first heart rate threshold.

With reference to the first aspect, in a possible implementation of the first aspect, the first time range may be determined in the following manner: determined based on the work and rest habit of the user, or determined based on time range information input by the user in a fourth user interface.

With reference to the first aspect, in a possible implementation of the first aspect, the second time range may be determined in the following manner:
determined based on the work and rest habit of the user, or determined based on time range information input by the user in a fourth user interface.

In other words, the first time range and the second time range may be determined based on the statistics collection on the work and rest habit of the user. For example, if work and rest of the user are that the user rests from 20:00 pm to 6:00 am on the next day and the user works from 6:00 am on the next day to 20:00 pm, the first time range may be from 20:00 pm to 6:00 am on the next day, and the second time range may be from 6:00 am on the next day to 20:00 pm.

Alternatively, the first time range and the second time range may be set by the user. For example, the user sets 9:00 am to 22:00 pm as the second time range, and sets 22:00 pm to 9:00 am on the next day as the first time range. This is not limited in this application.

With reference to the first aspect, in a possible implementation of the first aspect, the first preset time period may be determined in the following manner: determined based on the work and rest habit of the user, or determined based on time range information input by the user in a fourth user interface.

With reference to the first aspect, in a possible implementation of the first aspect, the second preset time period may be determined in the following manner: determined based on the work and rest habit of the user, or determined based on time range information input by the user in the fourth user interface.

According to a second aspect, an embodiment of this application further provides an electronic device. The electronic device includes a memory, storing computer program instructions; and a processor, where the processor is coupled to the memory. When the computer program instructions stored in the memory are executed by the processor, the electronic device is enabled to implement the blood pressure measurement method according to any one of the implementations of the first aspect.

According to a third aspect, an embodiment of this application provides a computer-readable storage medium. The computer-readable storage medium stores a computer program, and when the computer program is executed by a processor, the blood pressure measurement method according to any one of the implementations of the first aspect is implemented.

According to a fourth aspect, an embodiment of this application provides a computer program product. When the computer program product runs on an electronic device, the electronic device is enabled to perform the blood pressure measurement method according to any one of the implementations of the first aspect.

It may be understood that, for beneficial effects of the second aspect to the fourth aspect, refer to the related descriptions in the first aspect. Details are not described herein again.

### BRIEF DESCRIPTION OF DRAWINGS

To describe the technical solutions in embodiments of this application more clearly, the following briefly describes the accompanying drawings for describing embodiments or in the conventional technology. It is clear that the accompanying drawings in the following descriptions show merely some embodiments of this application, and persons of ordinary skill in the art may still derive other drawings from these accompanying drawings without creative efforts.
FIG. 1 is a diagram of a hardware structure of a wearable device for implementing a method in this application;
FIG. 2 is a diagram of determining a blood pressure measurement mode provided in a method in this application;
FIG. 3 is a diagram of determining a blood pressure measurement mode provided in a method in this application;
FIG. 4 is a diagram of determining a blood pressure measurement mode provided in a method in this application;
FIG. 5 is a diagram of determining a blood pressure measurement mode provided in a method in this application;
FIG. 6 is a diagram of determining a blood pressure measurement mode provided in a method in this application;
FIG. 7 is a diagram of determining a blood pressure measurement mode provided in a method in this application;
FIG. 8 is a diagram of determining a blood pressure measurement mode provided in a method in this application; and
FIG. 9 is a schematic flowchart of implementing a method in this application.

### DESCRIPTION OF EMBODIMENTS

The following describes aspects of illustrative embodiments by using terms commonly used by persons skilled in the art.

For ease of understanding this solution, some basic concepts and technical terms in this application are first described.

Blood pressure (blood pressure): lateral pressure acting on a side wall of a blood vessel per unit area when blood flows in the blood vessel. Blood pressure is a main driver of blood flow. Blood pressure of a human body needs to be kept within a normal fluctuation range, excessively high or low blood pressure has adverse effect on human health. Due to different blood vessels, blood pressure may be classified into arterial blood pressure (brachial artery), vein blood pressure, and capillary blood pressure. Generally, measured blood pressure is arterial blood pressure.

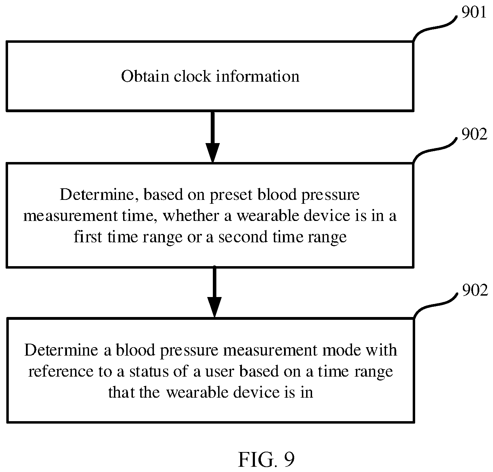

Oscillometric method: a method for measuring blood pressure. Specifically, a fluctuation envelope from a blood vessel wall is obtained through vibration caused by a collision of the blood vessel wall when the blood flows, and a blood pressure value is obtained by using a relationship between the fluctuation envelope and the arterial blood pressure. During specific measurement, an airbag and the like need to be bound to a limb of a person, and a method in which the airbag is used to compress an artery of a measured user, when blood pressure in the artery of the measured user hits the airbag, pressure fluctuation occurs in gas pressure inside the airbag, and then a blood pressure indicator of the measured user is calculated based on a fluctuation status of the gas pressure inside the airbag is used. This blood pressure measurement method is used to measure blood pressure of the user when the blood pressure is usually measured by using a wearable device. A specific implementation method is described below with reference to FIG. 9.

Corrected oscillometric method: A blood pressure measurement principle is the same as a pressure measurement principle of the oscillometric method, and pressure applied to a wrist of the user during blood pressure measurement is also the same. However, as described above, because there is a height difference between the wearable device on the wrist and the heart of the user when the user is in a lying posture, for example, the user lies flat or lies on the side, the height difference causes a change in a hydrostatic pressure difference between the brachial artery and the heart, causing a blood pressure change of the user. Generally, a proportion of blood in the human body is 1/13.6 of mercury. Therefore, each time a level distance between the brachial artery and the heart decreases by 1 cm, human blood pressure generally increases by 1/13.6 mmHg, that is, 0.735 mmHg. Therefore, when blood pressure measurement is performed on the user by using the corrected oscillometric method, a blood pressure value measured by using the oscillometric method is compensated by using the foregoing height difference, to obtain a final blood pressure measurement result. According to big data statistics, when the user is in the sleep state, the user is generally in a flat lying state. Therefore, in the implementations of this application, when it is detected that the user is in the sleep state, the user is in the flat lying state by default. In this case, compensation for a blood pressure difference caused by the height difference between a part of the user on which the wearable device is worn and the heart may be classified into two cases: In a first case, the user is in the flat lying state, and hands of the user are placed on the belly in an overlapping manner. In this case, a blood pressure value caused by the height difference between the part of the user on which the wearable device is worn and the heart is 3 to 5 mmHg less than a blood pressure value of the user measured by using the oscillometric method. In a second case, the user is in the flat lying state, and hands of the user are placed on a bed. In this case, a blood pressure value caused by the height difference between the part of the user on which the wearable device is worn and the heart is 3 to 5 mmHg greater than a blood pressure value of the user measured by using the oscillometric method. Therefore, it may be determined, based on a specific posture of the user during flat lying, to compensate the blood pressure value of the user measured by using the oscillometric method. A method for determining the posture of the user during flat lying is described below, and details are not described herein.

Dynamic blood pressure monitoring: also referred to as 24-hour dynamic blood pressure monitoring. It means that a blood pressure fluctuation change of the user within 24 hours is detected, and a blood pressure value obtained through measurement at an interval of specific time is referred to as dynamic blood pressure. In addition, based on a clinical blood pressure detection requirement, dynamic blood pressure monitoring is usually performed once every 15 to 20 minutes in the daytime, and a quantity of measured values needs to be greater than 20. However, during sleep at night, measurement is performed once every 30 minutes, and a quantity of measured values needs to be greater than 7. In this way, it can be ensured that data of the measured values meets the clinical blood pressure detection requirement without affecting sleep of the user, to provide a more professional and accurate blood pressure measurement result for the user. In an implementation of this application, this is used as an example for description. It may be understood that, in some implementations, when dynamic blood pressure monitoring is performed on the user, detection time intervals in the daytime and at night may alternatively be the same. For example, detection is performed once every 20 minutes in the daytime, and detection is also performed once every 20 minutes during sleep at night. This is not limited in this application.

The following describes a blood pressure measurement method in this application.

As described in the background, in a conventional dynamic blood pressure measurement method, regardless of whether the user is in the waking state or the sleep state, a pressurization function is usually automatically enabled, and the wrist of the user is pressurized to measure blood pressure of the user. In this manner, when the user is in the sleep state, especially when the user just falls asleep, the user is easily awakened, and sleep of the user is affected.

In addition, in a current solution, in a blood pressure measurement process, an upper limit value of pressure applied to the wrist of the user is controlled with reference to a status of the user. For example, for a same user, blood pressure of the user is 140 mmHg. When the user is in the waking state, the upper limit value of the pressure applied to the wrist of the user is 180 mmHg. When the user is in the sleep state, to avoid affecting the sleep state of the user, the upper limit value of the pressure applied to the wrist of the user is 150 mmHg. However, in one aspect, if the user just falls asleep and has light sleep, or the user usually has light sleep during napping in the daytime, even in a low-intensity pressurization mode (for example, in the sleep state of the user, pressure of 150 mmHg is used to measure blood pressure of the user), the user may be awakened, affecting sleep of the user. In another aspect, accuracy of blood pressure measurement in the low-intensity pressurization mode is generally lower than accuracy of blood pressure measurement in a normal-intensity pressurization mode. However, if the low-intensity pressurization mode is also used to obtain accuracy corresponding to the normal-intensity pressurization mode, a more advanced hardware facility is used, which undoubtedly increases manufacturing costs of the wearable device.

To prevent blood pressure measurement from affecting the sleep of the user and maintain accuracy of the blood pressure measurement result, this application provides a blood pressure measurement method.

It may be understood that, difficulty levels of interfering the user in different statuses by an external environment factor vary. Therefore, the statuses of the user may be classified into a sleep state, a suspected sleep state, and a waking state based on the difficulty levels of interfering the user by an external environment factor, and a sleep degree of the user, that is, whether the user is in a deep sleep state or a light sleep state, is further determined based on duration in which the user is in the sleep state. The user in the deep sleep state is difficult to be interfered by an external environment, and the user in the light sleep state is easy to be interfered by the external environment. The suspected sleep state is a state in which the user prepares for sleep. For example, the user lies flat on a bed and rests with eyes closed, and is also easy to be interfered by the external environment. Determining the sleep degree of the user based on the duration in which the user is in the sleep state is described below.

Based on this, in the blood pressure measurement method in this application, whether to perform blood pressure measurement on the user may be determined based on different statuses of the user. Specifically, when the user is in the waking state, blood pressure measurement may be performed on the user. When the user is in the deep sleep state, because it is difficult to be interfered by the external environment, blood pressure measurement may also be performed on the user in this case. When the user is in the light sleep state, because it is easy to be interfered by the external environment, blood pressure measurement may not be performed on the user in this case. When the user is in the suspected sleep state, to avoid affecting subsequent sleep of the user, blood pressure measurement is not performed on the user in this case. According to the foregoing method, impact of blood pressure measurement on the sleep status of the user can be reduced.

Further, as described above, when blood pressure of the user is measured, a height difference between a part (for example, a wrist) of the user on which a wearable device is worn and the heart needs to be as small as possible. Therefore, when blood pressure measurement is performed on the user, it is suggested to prompt the user to adjust a posture of the user to meet a blood pressure measurement requirement, so that a blood pressure measurement result is more accurate.

Specifically, in some implementations of this application, the user may be prompted when the user is in the waking state, so that the user adjusts the posture to meet the blood pressure measurement requirement. For example, the wearable device displays prompt information to prompt the user to raise the wrist to be level with the heart, and detects an angle between the wearable device and a horizontal plane by using a gyroscope sensor. When the angle between the wearable device and the horizontal plane meets a first condition, the blood pressure of the user is measured by using the oscillometric method. When the included angle between the wearable device and the horizontal plane does not meet a first condition, prompt information is sent to the user, to inform the user that current measurement is ineffective. The first condition is preset based on a specific posture used for blood pressure measurement. For example, if a blood pressure measurement posture requires the user to raise the wrist on which the wearable device is worn, so that the wearable device is level with the heart, an arm on which the wearable device is worn is closely attached to a user body, and a palm is placed on a shoulder, a preset angle condition is that the angle between the wearable device and the horizontal plane is greater than 60°. For ease of description, this mode is referred to as a first measurement mode in the following.

However, when the user is in the sleep state, the user is not prompted. In addition, when the user is in the sleep state, the user is lying flat by default. Therefore, in this case, a corresponding blood pressure measurement compensation value may be determined based on a specific lying flat posture of the user, for example, whether a hand of the user on which the wearable device is worn is placed on the belly of the user or on a bed. Specifically, because the included angle between the wearable device and the horizontal plane when the user places the hand on the belly is 10° to 20°, and the included angle between the wearable device and the horizontal plane when the user places the hand on the bed is 0° to 5°, the included angles between the wearable device and the horizontal plane in the two flat lying postures are different. Therefore, when the user is in the sleep state, the included angle between the wearable device and the horizontal plane may be detected by using the gyroscope sensor, to determine the specific flat lying posture of the user. Then, a blood pressure measurement value of the user that is obtained through measurement by using the oscillometric method is compensated based on the blood pressure compensation value corresponding to the specific flat lying posture of the user in the foregoing corrected oscillometric method. For ease of description, this mode is referred to as a second measurement mode in the following. A pressurization mode in the first measurement mode is the same as that in the second measurement mode. That is, regardless of whether the blood pressure of the user is measured in the first measurement mode or the second measurement mode, in a blood pressure measurement process, an upper limit value of pressure applied by the wearable device to the wrist of the user is related only to a blood pressure value of the user, and is unrelated to the status of the user.

In addition, in the second measurement mode, it may be understood that, in actual application, especially in clinical application, the foregoing dynamic blood pressure monitoring method is generally used to monitor a blood pressure change status of the user within 24 hours. In addition, the blood pressure of the user in the daytime is different from the blood pressure of the user at night, and the blood pressure of the user in the daytime is generally higher than the blood pressure at night. Therefore, when the dynamic blood pressure monitoring is used, there are different requirements on blood pressure measurement time intervals and quantities of measured values in the daytime and at night when it is ensured that impact on sleep of the user is low. For example, as described above, the blood pressure measurement time interval in the daytime is generally short, measurement is performed once every 15 to 20 minutes, and the quantity of measured values needs to be greater than or equal to 20. However, to reduce impact on sleep of the user at night, the blood pressure measurement time interval is long, measurement is performed once every 30 minutes, and the quantity of measured values needs to be greater than or equal to 7.

Therefore, to make the blood pressure measurement method in this application widely applicable to the clinical blood pressure monitoring field, in some implementations, the blood pressure measurement method in this application is further used to determine, based on different statuses of the user in the daytime and at night, to use the foregoing different blood pressure measurement modes to measure the blood pressure of the user. For a manner of determining to measure the blood pressure of the user by using different blood pressure measurement modes, refer to the foregoing related descriptions. Details are not described herein again.

A difference from the foregoing blood pressure measurement manner in which daytime and night are not distinguished lies in that the user is more likely to fall asleep at night than in the daytime, and the blood pressure measurement time interval at night is long. Therefore, when the user is in the sleep state at night, whether the user is in the deep sleep state may not be determined based on the duration in which the user is in the sleep state, but the user is in the deep sleep state by default after the user falls asleep at night. In other words, when the user is in the sleep state at night, the blood pressure of the user is measured in the second measurement mode.

In addition, based on big data statistics on cases in which most people are in the waking state at night, it can be learned that if being in the waking state at night, most people usually get up at night, work/entertain at night, or are insomnia. In these states, whether to perform blood pressure measurement on the user has different impact on sleep of the user. For example, the user continues to sleep after getting up at night. In this case, measuring the blood pressure of the user may affect subsequent sleep of the user, and when the user is insomnia, measuring blood pressure also affects subsequent sleep of the user.

Therefore, in an implementation of this application, night waking states of the user are further classified into a night get-up state, a night work/entertainment state, and an insomnia state based on impact on subsequent sleep of the user at night. Then, it is determined, based on different waking states of the user at night, to use the foregoing different blood pressure measurement modes to measure the blood pressure of the user.

Specifically, when the user is in the night get-up state or the insomnia state, measuring blood pressure of the user affects subsequent sleep of the user in this case. Therefore, blood pressure measurement is not performed on the user. When the user is in the night work/entertainment state, the blood pressure of the user is measured in a prompt measurement mode.

It can be learned that, in the foregoing method, the blood pressure of the user is measured in different blood pressure measurement modes with reference to different statuses of the user in the daytime and at night, so that impact of blood pressure measurement on the blood pressure of the user can be reduced, and a related requirement of clinical dynamic blood pressure monitoring is also met, to extend an application scope of the blood pressure measurement method in this application. The following uses this as an example for description.

For ease of description, in the blood pressure measurement method in this application, duration of one day (daytime and night) of the user is divided into a first time range (for example, 9:00 am to 20:00 pm) and a second time range (for example, 20:00 pm to 9:00 am on the next day) based on work and rest habits of most users. The first time range and the second time range may alternative be set by the user based on the work and rest habit of the user. For example, the user may set 10:00 am to 22:00 pm as the first time range, and set 22:00 pm to 10:00 am on the next day as the second time range. This is not limited in this application. In some implementations, the user may alternatively divide the daytime and the night into a plurality of time ranges based on a specific requirement. For example, the daytime is divided into two time ranges: 6:00 to 12:00 and 12:00 to 18:00, and the night is also divided into two time ranges: 18:00 to 22:00 and 22:00 to 6:00 on the next day. This is not limited in this application.

Then, in preset blood pressure measurement time, the blood pressure of the user is measured in different blood pressure measurement modes based on a time range and a status of the user. The preset blood pressure measurement time is dynamic blood pressure monitoring time, and start time of dynamic blood pressure monitoring may be set by the user. For example, the user may set the start time of dynamic blood pressure monitoring to 00:00. Then, dynamic blood pressure monitoring is performed once every 20 minutes in the first time range, and is performed once every 30 minutes in the second time range. This is not limited in this application. In some other implementations, when a dynamic blood pressure monitoring measured value requirement is met, measurement time intervals of the dynamic blood pressure monitoring in different time ranges may alternatively be set by the user based on a specific case. This is not limited in this application.

Specifically, when the user is in the waking state in the first time range, the blood pressure of the user is measured in the first measurement mode. When the user is in the deep sleep state in the first time range, the blood pressure of the user is measured in the second measurement mode. When the user is in a non-deep sleep state in the first time range, the blood pressure of the user is not measured. When the user is in the sleep state in the second time range, as described above, the user is in the deep sleep state by default, that is, the blood pressure of the user is measured in the second measurement mode. When the user is in the waking state in the second time range, as described above, a manner of measuring the blood pressure of the user is further determined based on that the user is in the night get-up state, the night work/entertainment state, or the insomnia state. If the user is in the night get-up state or the insomnia state, to avoid affecting subsequent sleep of the user, the blood pressure of the user is not measured. If the user is in the night work/entertainment state, the blood pressure of the user is measured in the first measurement mode. However, when the user is in the suspected sleep state, the blood pressure of the user is not measured regardless of a range in which the user is.

More specifically, for example, it is detected in the preset blood pressure measurement time that the user is in the waking state in the first time range (9:00 to 20:00). Because the user is in the waking state from 9:00 to 20:00, in this case, the blood pressure of the user is measured in the first measurement mode. In some implementations, a prompt manner includes but is not limited to a manner of performing prompt through vibration, voice broadcast, or by sending prompt information to the user. This is not limited in this application.

For another example, it is detected in the preset blood pressure measurement time that the user is in the waking state in the second time range (20:00 to 9:00), it may be inferred that the user may be in the night get-up, insomnia, or night work/learning/entertainment state or the like. Therefore, there is a need to further determine whether the user is in the night get-up or insomnia scenario, or in the night work/learning/entertainment state or the like, and then a specific blood pressure measurement mode for measuring the blood pressure of the user is determined with reference to the status of the user. If the user is in the night get-up state, because the user enters the sleep state again after the user gets up at night, blood pressure measurement on the user affects subsequent sleep of the user in this case. In addition, movement of the user fluctuates greatly during getting up at night, and a result of blood pressure measurement on the user may be inaccurate in this case. Therefore, when the user is in the night get-up state, blood pressure measurement is not performed on the user. If the user is in the insomnia state, to avoid aggravating impact on sleep of the user, blood pressure measurement is not performed on the user. If the user is in the night work/learning/entertainment state or the like, the blood pressure of the user is measured in the first measurement mode. Specifically, in some implementations, if the user is in the night work/learning/entertainment state or the like, before performing blood pressure measurement on the user, the wearable device may display prompt information "Please confirm that the wrist position is level with the heart" to prompt the user to adjust the blood pressure measurement posture, and after the user taps a corresponding confirmation button and confirms that the posture adjustment is completed, the wearable device continues to perform blood pressure measurement on the user.

For another example, it is detected in the preset blood pressure measurement time that the user is in the sleep state in the first time range (9:00 to 20:00). As described above, the user has light sleep in the daytime, and is prone to external interference. Therefore, there is a need to further determine whether time for which the user is in the sleep state is greater than a first preset value, to determine whether the user is in the deep sleep state. When the time for which the user is in the sleep state exceeds the first preset value, it indicates that the user enters the deep sleep state. In this case, the blood pressure of the user may be measured in the second measurement mode. The first preset value is an empirical value or an experimental value. For example, the first preset value may be 1.5 hours.

For another example, when it is detected in the preset blood pressure measurement time that the user is in the sleep state in the second time range (20:00 to 9:00 on the next day), in one aspect, as described above, the measurement interval (30 minutes) of the second time range is greater than the measurement interval (20 minutes) of the second time range, and the duration (9:00 to 20:00) of the first time range is less than the duration (20:00 to 9:00) of the second time range. Therefore, if it is still determined whether the duration in which the user is in the sleep state exceeds the first preset value, a quantity of pieces of obtained night measurement data may not meet the quantity 7 or more used for the clinical dynamic blood pressure monitoring. In another aspect, from a physiological perspective, the user is more likely to sleep at night than in the daytime. For example, the user body secretes melatonin at night for sleep, and is more difficult to sleep in the daytime due to interference of an environment or human factors such as noise and ambient light. Therefore, when it is detected that the user is in the sleep state in the second time range, it is unnecessary to determine whether the duration in which the user is in the sleep state is greater than the first preset value, but the blood pressure of the user is measured in the second measurement mode.

For another example, it is detected in the preset blood pressure measurement time that the user is in the suspected sleep state in the first time range (9:00 to 20:00) or the second time range (20:00 to 9:00 on the next day). Because the user may be sleeping, for example, lying flat and resting, blood pressure measurement is not performed on the user in this case to avoid affecting sleep of the user.

According to the foregoing method, accuracy of the pressure measurement result can be ensured without affecting sleep of the user, and the clinical dynamic blood pressure monitoring requirement can also be met, so that an accurate and professional blood pressure measurement result is provided for the user, to improve user experience.

The blood pressure measurement method may be applied to the wearable device such as a smartwatch or a smart band, or any device. The wearable device includes but is not limited to a portable terminal device on which iOS^{™}, Android^{™}, Microsoft^{™}, Harmony^{™}, or another operating system is installed. A specific type of the wearable device is not limited in this application.

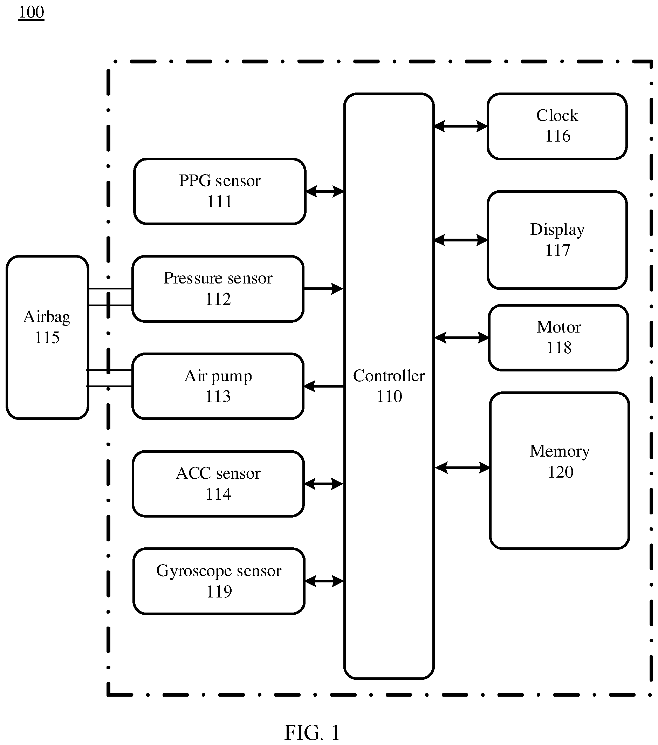

FIG. 1 is a diagram of a hardware structure of a wearable device according to an embodiment of this application.

As shown in FIG. 1, a structure of the wearable device 100 includes a controller 110, a photoplethysmography (photoplethysmography, PPG) sensor 111, a pressure sensor 112, an air pump 113, an accelerometer (accelerometer, ACC) sensor 114, an airbag 115, a clock 116, a display 117, a motor 118, a gyroscope sensor 119, and a memory 120.

In some implementations, the PPG sensor 111 and/or the ACC sensor 114 may form a sleep monitoring module, configured to monitor a status of a user, that is, whether the user is in a waking state, a suspected sleep state, or a sleep state.

The PPG sensor 111 is configured to collect a heart rate of the user, to determine the status of the user based on the heart rate of the user. For example, the heart rate of the user in the waking state is generally 70 times/minute. When the user is in the sleep state, the heart rate decreases by 10 to 15 times/minute, and changes to 55 times/minute to 60 times/minute. When the user is in the suspected sleep state, the heart rate of the user is between the two. Therefore, in some implementations, a state corresponding to the heart rate from 55 times/minute to 60 times/minute may be set to the sleep state, a state corresponding to the heart rate not less than 70 times/minute may be set to the waking state, and a state corresponding to the heart rate from 60 times/minute to 70 times/minute may be set to the suspected sleep state. It may be understood that the heart rate has an individual difference. Therefore, in some implementations, the foregoing states may alternatively be set based on a heart rate and physiological indicator data such as a work and rest status of a specific user and the corresponding heart rate, so that a correspondence between a heart rate and a status of the user can be more suitable for an actual situation of the specific user. This is not limited in this application.

It may be understood that, in some implementations, a normal sinus rhythm interval sequence and a breathing signal may also be extracted from a heart rate signal collected by the PPG sensor 111, correlation and cross-spectrum power of the two signals are analyzed by using a Hilbert-Huang transform (Hilbert-Huang Transform, HHT) technology, and then a cardiopulmonary coupling (Cardiopulmonary Coupling, CPC) dynamics spectrum during sleep is generated, to determine the status of the user. This is not limited in this application.

The ACC sensor 114 is configured to collect acceleration data of the user, to determine whether the user has continuous motion, that is, whether the user is in a continuous motion state, so as to further determine the status of the user. For example, when it is detected that an acceleration value of the user is not 0 or is greater than a preset acceleration threshold in a time period, it indicates that the user is in the continuous motion state, and it may also be determined that the user is in the waking state in this case. When it is detected that an acceleration value of the user is 0 or is less than a preset acceleration threshold in a time period (for example, 20 minutes), it may indicate that the user is not in the continuous motion state, that is, the user may be in the sleep state or the suspected sleep state. In this case, the status of the user may be further determined with reference to the heart rate of the user collected by the PPG sensor 211. If the heart rate of the user collected by the PPG sensor 111 is less than 60 times/minute in this case, it may be determined that the user is in the sleep state. If the heart rate of the user collected by the PPG sensor 111 is greater than 60 times/minute in this case, it may be determined that the user is in the suspected sleep state.

The air pump 113, the airbag 115, and the pressure sensor 112 establish a communication connection to the controller 110 by using a system bus, and the airbag 115 is connected to the air pump 113 and the pressure sensor 112. When the wearable device 100 controls the air pump 113 to inflate and deflate the airbag 115, the wearable device 100 may compress a blood vessel, collect a pulse wave signal by using the pressure sensor 112, and then fit a blood pressure value of the user by using the pulse wave signal. In some implementations, corresponding to a first measurement mode, after detecting a touch operation that the user confirms that a blood pressure measurement posture is adjusted, the wearable device 100 controls the air pump 113 to perform a first measurement operation on the airbag 115, that is, controls the air pump 113 to inflate and deflate the airbag 115, to compress the blood vessel and collect the pulse wave signal by using the pressure sensor 112, and then fits a first blood pressure value of the user. In some other implementations, corresponding to a second measurement mode, the wearable device 100 controls the air pump 113 to perform a second measurement operation on the airbag 115, that is, controls the air pump 113 to inflate and deflate the airbag 115, to compress the blood vessel and collect the pulse wave signal by using the pressure sensor 112, and then calculates a second blood pressure value of the user with reference to a blood pressure compensation value corresponding to an included angle that is between the wearable device 100 and a horizontal plane and that is detected by the gyroscope sensor 119. The first measurement operation and the second measurement operation correspond to a same pressurization mode. The same pressurization mode means that an upper limit value of pressure applied to a wrist of the user is related only to the blood pressure value of the user, and is unrelated to the status of the user. The clock 116 is configured to obtain clock information. In this embodiment of this application, the wearable device 100 may determine, by obtaining the clock information, whether a current moment is a first time range or a second time range, to further determine the sleep state of the user with reference to the PPG sensor 111 and the ACC sensor 114, and then perform blood pressure measurement on the user in different blood pressure measurement modes based on a time range and the sleep state of the user.

The gyroscope sensor 119 is configured to detect an included angle between an arm of the user on which the wearable device 100 is worn and the horizontal plane, to subsequently determine, by using the included angle, a manner of performing blood pressure value compensation on the user. In some implementations, the gyroscope sensor 119 may be further configured to detect, in the first measurement mode, whether the included angle between the wearable device 100 and the horizontal plane meets a first condition, so that the wearable device 100 can determine, based on a determining result, whether current measurement is ineffective.

The controller 110 may be a central processing unit (central processing unit, CPU). The controller 110 may determine, with reference to the time range and the status that are of the user and that are determined by the PPG sensor 111, the ACC sensor 114, and the clock 116, a specific blood pressure measurement mode for measuring blood pressure of the user, and control, based on the specific blood pressure measurement mode, the air pump 113 to inflate the airbag 115 or deflate the airbag 115.

The memory 120 may include a volatile memory (volatile memory), such as a random-access memory (random-access memory, RAM); the memory may also include a non-volatile memory (non-volatile memory), such as a read-only memory (read-only memory, ROM), a flash memory, a hard disk drive (hard disk drive, HDD), or a solid-state drive (solid-state drive, SSD); or the memory may further include a combination of the foregoing types of memories. In some other implementations, the memory 120 may further record a moment at which the user enters the sleep state each time and a moment at which the user enters the waking state, to determine, based on the moment at which the user enters the sleep state, duration in which the user enters the sleep state, or determine, based on the moment at which the user enters the waking state, duration in which the user enters the waking state.

It may be understood that the structure shown in FIG. 1 is merely an example, and does not constitute a specific limitation on a hardware structure of the wearable device 100. In another embodiment, the wearable device 100 may further include more hardware, such as a pressure sensor, a speaker, and a gyroscope sensor.

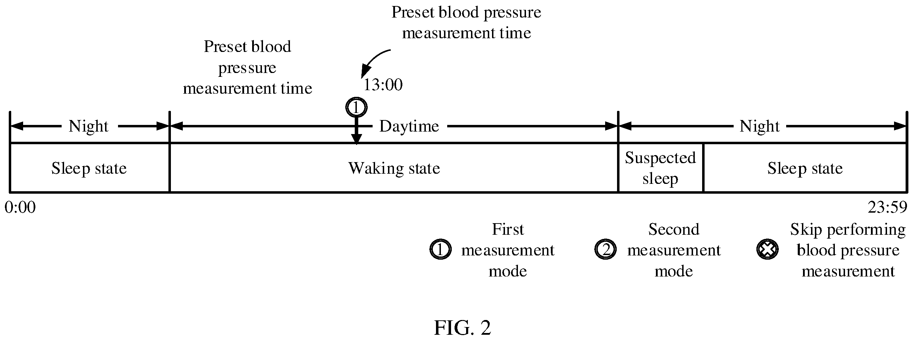

The following describes a specific implementation process of the blood pressure measurement method in this application with reference to FIG. 2 to FIG. 8.

As described above, preset blood pressure measurement time may be that starting from 00:00, measurement is performed once every 20 minutes in a first time range, and measurement is performed once every 30 minutes in a second time range.

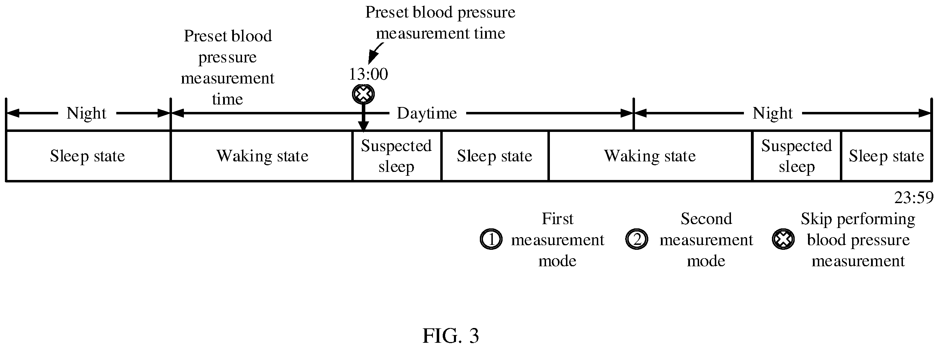

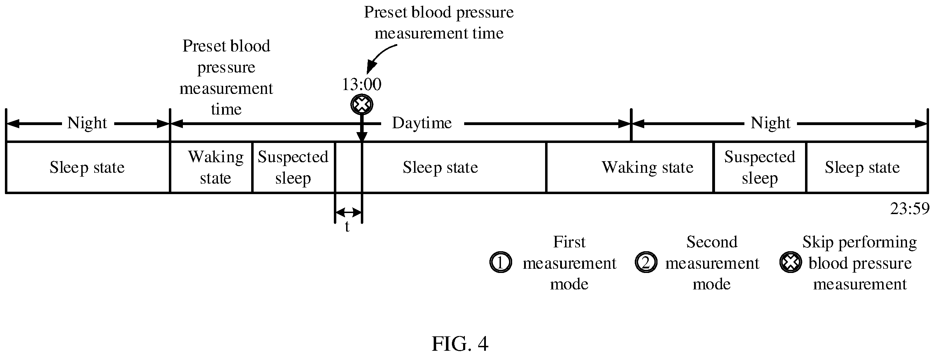

For example, it is assumed that preset blood pressure measurement time is 13:00. After the preset blood pressure measurement time arrives, the wearable device 100 detects a current status of a user. If the user is in a waking state, as shown in FIG. 2, blood pressure of the user is measured in a first measurement mode. If the user is in a suspected sleep state, as shown in FIG. 3, to avoid affecting subsequent sleep of the user, blood pressure of the user is not measured. As shown in FIG. 4, if the user is in a sleep state, it is further determined whether duration in which the user is in the sleep state is greater than a first preset value, that is, it is determined whether duration t from a moment at which the user enters the sleep state this time to the preset blood pressure measurement time is greater than the first preset value. When it is determined that the duration from the moment at which the user is in the sleep state to the preset blood pressure measurement time is greater than the first preset value, it indicates that the user may enter a deep sleep state, and in this case, blood pressure of the user is measured in a second measurement mode. When it is determined that the duration from the moment at which the user is in the sleep state to the preset blood pressure measurement time is less than or equal to the first preset value, it indicates that time for which the user enters the sleep state is short, that is, the user may not enter the deep sleep state. For example, the user only takes a nap in a work interval. In this case, to avoid disturbing sleep of the user, blood pressure of the user is not measured. When calculating the duration in which the user is in the sleep state, the wearable device 100 may obtain, from the memory 120, the moment at which the user enters the sleep state this time, and duration between the current moment and the moment at which the user enters the sleep state this time is the duration in which the user enters the sleep state this time.

For example, it is assumed that the wearable device 100 detects that the moment at which the user enters the sleep state is 12:30, and the duration t ending at the preset blood pressure measurement time 13:00 is 0.5 hour and is less than the first preset value (1.5 hours). Therefore, it indicates that the user has not entered the deep sleep state, and therefore blood pressure of the user is not measured. When next preset blood pressure measurement time 13:20 arrives, it continues to detect whether the duration t in which the user is in the sleep state exceeds the first preset value in this case. If the duration t does not exceed the first preset value, the blood pressure of the user is still not measured. Each time the preset blood pressure measurement time arrives subsequently, it is determined whether the duration in which the user is in the sleep state exceeds the first preset value, until the duration exceeds the first preset value, the blood pressure of the user is measured in the second measurement mode. If the duration in which the user is in the sleep state does not exceed the first preset value, the blood pressure of the user is not measured.

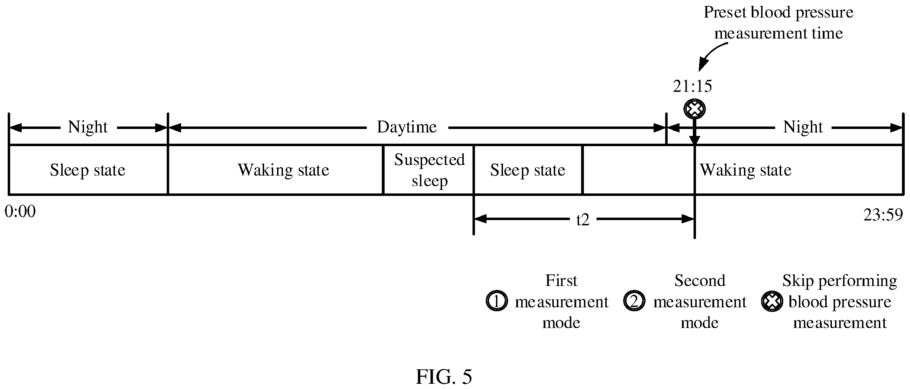

For another example, it is assumed that preset blood pressure measurement time is 21:15. A status of the user is detected when the preset blood pressure measurement time arrives. If it is detected that the user is in the waking state at the preset blood pressure measurement time, it is further determined whether duration t2 of the user from the latest sleep state is greater than a second preset value, that is, whether the duration t2 between the preset blood pressure measurement time and a moment at which the user enters the sleep state last time is greater than the second preset value. If the duration t2 is greater than the second preset value, as shown in FIG. 5, it indicates that the user has been in the waking state for a long time at night, that is, the user may be in a scenario such as night work/learning/entertainment, or in a night insomnia state. If the user is in the night work/learning/entertainment state or the like, the blood pressure of the user is measured in the first measurement mode. If the user is in the night insomnia state, to avoid aggravating impact of blood pressure measurement on sleep of the user, the blood pressure of the user is not measured. In some implementations, the second preset value is an empirical value or an experimental value. For example, the second preset value may be 10 hours.

In some implementations, specifically, the user may preset the night work/entertainment state or the insomnia state of the user based on a work and rest habit of the user.

For example, if the user often works or entertains in a time period at night, the user may set the time period as a work or entertainment time period in a corresponding operation setting interface when setting preset blood pressure measurement start time by using the wearable device. In this way, when detecting that the user is also in the work or entertainment time period at the preset blood pressure measurement time (21:15), the wearable device 100 may determine that the user is in the night work/entertainment state, and measure the blood pressure of the user in the first measurement mode.

If the user is often insomnia in a time period at night, the user may set the time period as an insomnia time period in a corresponding operation setting interface when setting preset blood pressure measurement start time by using the wearable device. In this way, when detecting that the user is also in the insomnia time period at the preset blood pressure measurement time (21:15), the wearable device 100 determines that the user is in the insomnia state, and does not measure the blood pressure of the user. This is not limited in this application.

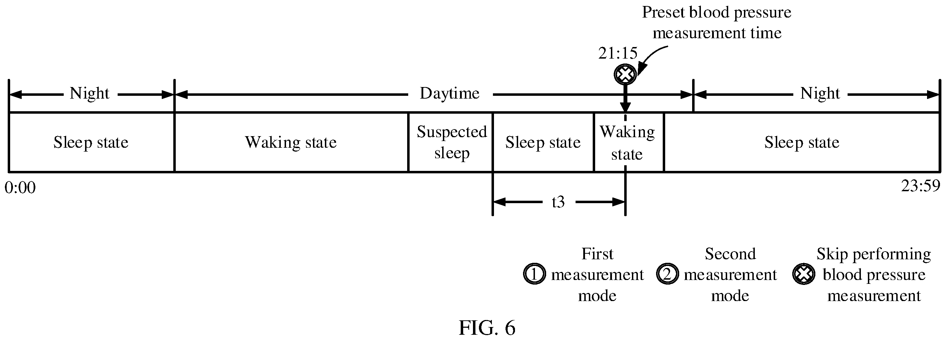

If it is detected that the user is in the waking state at the preset blood pressure measurement time, but it is also detected that duration t3 in which the user is in the waking state is less than a third preset value, as shown in FIG. 6, it indicates that the user may be in a night get-up scenario, that is, the user is only awake for a short period of time. In one aspect, the user continues to rest after getting up at night, if the blood pressure of the user is measured in this case, sleep of the user is affected. In another aspect, the user is also in an active state when getting up at night, measurement on the blood pressure of the user may be inaccurate in this case. Therefore, when it is detected that the duration t3 in which the user is in the waking state at the preset blood pressure measurement time is less than the third preset value, the blood pressure of the user is not measured. In some implementations, the third preset value is an empirical value or an experimental value. For example, the third preset value may be 0.1 hour.

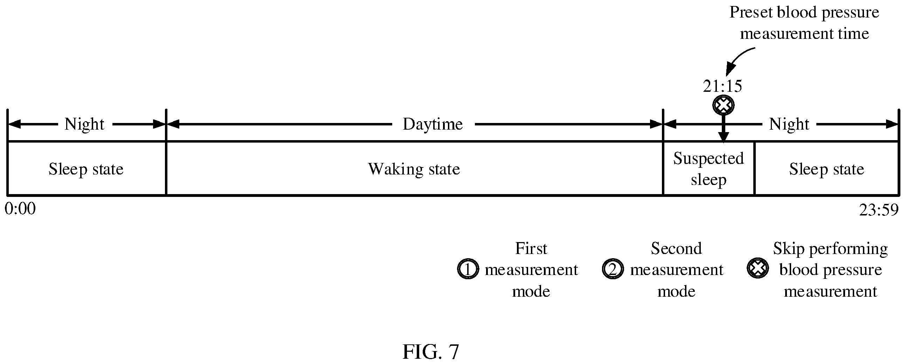

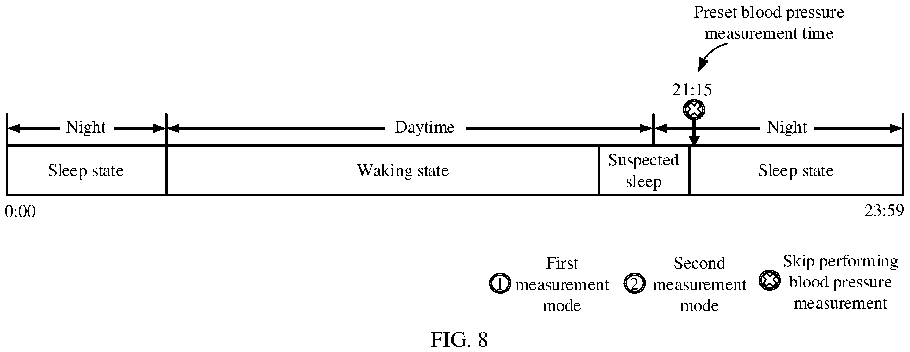

If it is detected that the user is in the suspected sleep state at the preset blood pressure measurement time, as shown in FIG. 7, the blood pressure of the user is not measured. If it is detected that the user is in the sleep state at the preset blood pressure measurement time, as shown in FIG. 8, as described above, the user is more likely to enter the deep sleep state at night than in the daytime, or in other words, the user can enter the sleep state more quickly than in the daytime. Therefore, the blood pressure of the user may be measured in the second measurement mode in this case.

For ease of understanding the foregoing implementation process, the following describes specific implementation details of the blood pressure measurement method in this application with reference to FIG. 9. The method is implemented by a wearable device 100. It may be understood that, in another embodiment, the method may alternatively be implemented by the wearable device 100 in cooperation with another electronic device such as a smartphone. For example, the smartphone obtains a status and a time range that are of a user and that are monitored by the wearable device 100, and then the smartphone controls a specific blood pressure measurement mode used by the wearable device 100 to measure blood pressure of the user. This is not limited in this application.

901: Obtain clock information.

The wearable device 100 may send the obtained clock information to a controller 110 based on the clock information of a clock 116.

In some embodiments, the wearable device 100 reads local time in the clock 116 to obtain the clock information, and sends the obtained clock information to the controller 110. It may be understood that the local time may be set by the user. In some embodiments, in a running process of the wearable device 100, a specific quantity of offsets may be generated in the local time. As a result, it may not be ensured that obtained time is accurate and stable by relying only on the local time. Therefore, the wearable device 100 may further have a wireless communication module, and obtain accurate network time from an electronic device in the wireless communication module as accurate clock information. The wireless communication module may be, for example, Bluetooth or near field communication (near field communication, NFC). This is not limited in this application. In addition, the clock information may alternatively be obtained in any other manner such as manual input or automatic calibration in a calculation manner. This is not limited herein. In some embodiments, the clock information may be a global positioning system (global positioning system, GPS) time service or the like.

902: Determine, based on preset blood pressure measurement time, whether the wearable device is in a first time range or a second time range. The preset blood pressure measurement time, the first time range, and the second time range may all be manually set by the user. For example, based on a work and rest habit of the user, the user sets 9:00 to 20:00 as the first time range, and sets 20:00 to 00:00 and 00:00 to 9:00 as the second time range. Then, the user sets a dynamic blood pressure measurement start time to 00:00, measurement is performed once every 20 minutes in the first time range, and measurement is performed once every 30 minutes in the second time range, to ensure that effective measurement values in the first time range and the second time range meet the foregoing clinical dynamic blood pressure measurement requirement (a quantity of effective values in the daytime is greater than or equal to 20, and a quantity of effective values at night is greater than or equal to 7) without frequently affecting work or rest of the user.

In some embodiments, the first time range and the second time range may alternatively be other time ranges. For example, 7:00 to 22:00 is the first time range, and 22:00 to 00:00 and 00:00 to 7:00 are the second time range. Correspondingly, to ensure that effective values in each time range meet the foregoing clinical dynamic blood pressure measurement requirement, the preset blood pressure measurement time and the time interval may alternatively be adjusted based on a requirement. This is not limited in this application.

After the first time range (for example, 9:00 to 20:00), the second time range, the dynamic blood pressure measurement start time (00:00), and the time interval (measurement is performed once every 20 minutes in the first time range, and measurement is performed once every 30 minutes in the second time range) are set, a time range in which the user is currently is determined based on the preset blood pressure measurement time. For example, starting from 00:00, it is assumed that preset blood pressure measurement time during the period is 00:30, and it is determined, based on the time, that a time range in which the user is at 00:30 is the second time range. For another example, starting from 00:00, it is assumed that preset blood pressure measurement time during the period is 9:50, and it is determined, based on the time, that a time range in which the user is at 9:50 is the first time range.

903: Determine a blood pressure measurement mode with reference to a status of the user based on the time range that the wearable device is in. As described above, in different time ranges, dynamic blood pressure measurement time intervals are different, and difficulty levels of entering deep sleep by the user are also different. Therefore, even if the user is in a same state in the first time range and the second time range, different blood pressure measurement modes may need to be used to measure the blood pressure of the user, to reduce impact on sleep of the user. For a specific method for determining the blood pressure measurement mode with reference to the status of the user and the time range in which the user is, refer to related descriptions in FIG. 2 to FIG. 8. Details are not described herein again.

Specifically, in some embodiments, a manner of measuring the blood pressure of the user may be a linear boost manner. To be specific, the controller 110 of the wearable device 100 controls an air pump, so that the air pump 113 pressurizes an airbag 115 to a first gas pressure value (unit: millimeter of mercury (mmHg)), and then the controller 110 obtains a pulse signal collected by a pressure sensor 112, and determines the blood pressure of the user based on the pulse signal. In some embodiments, the first gas pressure value may be [160 mmHg, 200 mmHg], for example, 170 mmHg. In some embodiments, the air pump 113 is used to pressurize and restart the airbag 115, so that internal pressure of the airbag 115 is linearly boosted at a specific rate (for example, 3 mmHg to 6 mmHg). In addition, the pressure sensor 112 connected to the airbag 115 detects the internal pressure of the airbag 115, and obtains a pressure signal. Then, a static pressure signal and a pulse wave signal are extracted from the pressure signal. Systolic pressure and diastolic pressure are obtained through calculation based on the extracted static pressure signal and pulse wave signal. Persons skilled in the art should be noted that a manner of calculating the systolic pressure and the diastolic pressure by using the static pressure signal and the pulse wave signal is the same as a calculation manner in an existing solution, and details are not described herein. When gas pressure in the airbag 115 is pressurized to some extent, all features of the pulse wave signal are extracted. The wearable device 100 may control the air pump 113 to stop pressurizing and perform deflating. In this case, a blood pressure measurement process ends.

In some implementations, in a pressurization process, the pulse wave signal and the static pressure signal that are extracted from the original pressure signal may be to extract one or more feature points in a large pulse wave signal, and the static pressure signal may be a static pressure value. The static pressure value is a static pressure value corresponding to a feature point in the pulse wave signal. To extract all feature information of the pulse wave signal, pressure is usually increased to a high level. For example, 20 mmHg to 40 mmHg higher than systolic pressure (99 mmHg to 119 mmHg) of a normal human body is applied, for example, 170 mmHg is applied.

The following describes a method for determining statuses of the user in different time ranges.

As described above, when the user is in different statuses, values of the heart rate of the user are different, and when the user is in the sleep state and the waking state, the user has different motion statuses. Therefore, the status of the user may be determined by detecting the heart rate of the user by using the PPG sensor 111 and/or detecting the motion status of the user by using the ACC sensor 114.

Specifically, in some implementations, the heart rate of the user may be detected by using the PPG sensor 111, and then the status of the user is determined based on the foregoing preset mapping relationship between the heart rate of the user and the status of the user.

For example, when the heart rate of the user is greater than the first heart rate threshold, it is determined that the user is currently in the waking state. When the heart rate of the user is less than the second heart rate threshold, it is determined that the user is currently in the sleep state. When the heart rate of the user is greater than the third heart rate threshold and less than the fourth heart rate threshold, it is determined that the user is in the suspected sleep state. The first heart rate threshold, the second heart rate threshold, and the third heart rate threshold are all experimental values or empirical values. A value of the first heart rate threshold may be, for example, 70 times/minute. A value of the second heart rate threshold may be, for example, 60 times/minute. The third heart rate threshold may be less than or equal to the second heart rate threshold, for example, a value of the third heart rate threshold may be 55 times/minute. The fourth heart rate threshold may be greater than or equal to the second heart rate threshold, for example, may be 60 times/minute. This is not limited in this application.

In some other implementations, the status of the user may alternatively be determined based on an acceleration value of the user detected by the ACC sensor 114. For example, when the ACC sensor 114 detects that continuous motion occurs on a body part of the user on which the wearable device 100 is worn, it may be determined that the user is in the waking state. For the suspected sleep state and the sleep state, because motion statuses corresponding to the two are similar, in this case, as described above, whether the user is in the suspected sleep state may be jointly determined with reference to the heart rate of the user detected by the PPC sensor 111. For example, when the ACC sensor 114 does not detect that the user has continuous motion, and the heart rate of the user detected by the PPG sensor 111 is greater than the third heart rate threshold and less than the fourth heart rate threshold, it may be determined that the user is in the suspected sleep state. When the ACC sensor 114 does not detect that continuous motion occurs on the body part of the user, and the heart rate of the user detected by the PPG sensor 111 is less than the second heart rate threshold, it may be determined that the user is in the sleep state.

An embodiment of this application further provides an electronic device. The electronic device includes: at least one processor, a memory, and a computer program that is stored in the memory and that is executable on the at least one processor. When executing the computer program, the processor implements the steps in any one of the foregoing method embodiments.

An embodiment of this application further provides a computer-readable storage medium. The computer-readable storage medium stores a computer program. When the computer program is executed by a processor, the steps in the foregoing method embodiments can be implemented.

An embodiment of this application provides a computer program product. When the computer program product runs on a mobile terminal, the steps in the foregoing method embodiments can be implemented by the mobile terminal.

When the integrated unit is implemented in the form of the software functional unit and sold or used as an independent product, the integrated unit may be stored in a computer-readable storage medium. Based on such an understanding, in this application, all or some of the procedures of the methods in the foregoing embodiments may be implemented by a computer program instructing related hardware. The computer program may be stored in a computer-readable storage medium. When the computer program is executed by a processor, the steps in the method embodiments can be implemented. The computer program includes computer program code, and the computer program code may be in a source code form, an object code form, an executable file form, an intermediate form, or the like. The computer-readable medium may include at least any entity or apparatus that can carry computer program code to a photographing apparatus/terminal device, a recording medium, a computer memory, a read-only memory (read-only memory, ROM), a random access memory (random access memory, RAM), an electrical carrier signal, a telecommunication signal, and a software distribution medium, for example, a USB flash drive, a removable hard disk, a magnetic disk, or an optical disk. In some jurisdictions, the computer-readable medium cannot be an electrical carrier signal or a telecommunication signal according to legislation and patent practices.

In the foregoing embodiments, descriptions of all embodiments have respective focuses. For a part that is not described in detail in an embodiment, refer to related descriptions in another embodiment.

Persons of ordinary skill in the art may be aware that, in combination with the examples described in embodiments disclosed in this specification, units and algorithm steps can be implemented by electronic hardware or a combination of computer software and electronic hardware. Whether the functions are performed by hardware or software depends on particular applications and design constraint conditions of the technical solutions. Persons skilled in the art may use different methods to implement the described functions for each particular application, but it should not be considered that the implementation goes beyond the scope of this application.

In embodiments provided in this application, it should be understood that the disclosed apparatus/network device and method may be implemented in other manners. For example, the described apparatus/network device embodiment is merely an example. For example, division into the modules or units is merely logical function division and may be other division during actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electrical, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In the foregoing descriptions, for illustration instead of limitation, specific details such as a particular system structure and a technology are provided to make a thorough understanding of embodiments of this application. However, persons skilled in the art should understand that this application may also be implemented in other embodiments without these specific details. In other cases, detailed descriptions of well-known systems, apparatuses, circuits, and methods are omitted, so that this application is described without being obscured by unnecessary details.

It should be understood that, when used in this specification and the appended claims of this application, the term "include" indicates presence of the described features, entireties, steps, operations, elements, and/or components, but does not exclude presence or addition of one or more other features, entireties, steps, operations, elements, components, and/or sets thereof.

It should be further understood that the term "and/or" used in this specification and the appended claims of this application indicates any combination and all possible combinations of one or more items listed in association, and includes the combinations.

As used in this specification and the appended claims of this application, based on the context, the term "if" may be interpreted as "when" or "once" or "in response to determining" or "in response to detecting". Likewise, the phrase "if it is determined that" or "if (a described condition or event) is detected" may be interpreted as a meaning of "once it is determined that" or "in response to determining" or "once (a described condition or event) is detected" or "in response to detecting (a described condition or event)" depending on the context.

In addition, in the descriptions of this specification and appended claims of this application, the terms "first", "second", "third", and the like are merely intended for the purpose of differentiated description, but shall not be understood as an indication or an implication of relative importance.

Reference to "an embodiment", "some embodiments", or the like described in this specification of this application indicates that one or more embodiments of this application include a specific feature, structure, or characteristic described with reference to the embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner.

The foregoing embodiments are merely intended to describe the technical solutions of this application, but are not to limit this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application, and these modifications and replacements shall fall within the protection scope of this application.

## Claims

1. A blood pressure measurement method, applied to a wearable device, wherein the wearable device comprises a photoplethysmography PPG sensor, a micropump, and an airbag, and the method comprises:
determining a heart rate of a user based on the PPG sensor;
if the heart rate of the user is greater than a first heart rate threshold, displaying a first user interface, wherein the first user interface is used to prompt the user to measure blood pressure;
in response to a detected first operation performed on the first user interface, controlling the micropump to perform a first measurement operation on the airbag, wherein the first measurement operation is used to measure the blood pressure;
if the heart rate of the user is less than a second heart rate threshold, determining whether a current moment is in a first time range, wherein the second heart rate threshold is less than or equal to the first heart rate threshold; and
if the current moment is in the first time range, controlling the micropump to perform a second measurement operation on the airbag, wherein the second measurement operation is used to measure the blood pressure, wherein
a same pressurization mode is used for the first measurement operation and the second measurement operation.

2. The method according to claim 1, wherein the wearable device further comprises a gyroscope sensor and a pressure sensor, the pressure sensor is configured to detect pressure of the airbag, and the method further comprises:
in a process of the first measurement operation, determining a first signal based on the gyroscope sensor, and determining a second signal based on the pressure sensor; and
if the first signal meets a first condition, displaying a second user interface after the first measurement operation, wherein the second user interface comprises a first blood pressure value, and the first blood pressure value is determined based on the second signal; or
if the first signal does not meet the first condition, displaying a third user interface, wherein the third user interface is used to inform the user that measurement is ineffective.

3. The method according to claim 2, wherein the third user interface is displayed after the first measurement operation ends.

4. The method according to any one of claims 1 to 3, wherein the wearable device further comprises the gyroscope sensor and the pressure sensor, the pressure sensor is configured to detect the pressure of the airbag, and the method further comprises:
in a process of the second measurement operation, determining a third signal based on the gyroscope sensor, and determining a fourth signal based on the pressure sensor; and
determining a second blood pressure value based on the third signal and the fourth signal after the second measurement operation.

5. The method according to claim 1, wherein the method further comprises:
if the heart rate of the user is less than the second heart rate threshold, the current moment is in the first time range, and duration in which the heart rate of the user is less than the second heart rate threshold is greater than first preset duration, controlling the micropump to perform the second measurement operation on the airbag, wherein the second measurement operation is used to measure the blood pressure.

6. The method according to claim 1, wherein the method further comprises:
if the heart rate of the user is greater than the first heart rate threshold, the current moment is in the first time range, and the current moment is in a first preset time period, displaying the first user interface, wherein the first user interface is used to prompt the user to measure the blood pressure; and
in response to the detected first operation performed on the first user interface, controlling the micropump to perform the first measurement operation on the airbag.

7. The method according to claim 1, wherein the method further comprises:
if the heart rate of the user is greater than the first heart rate threshold, the current moment is in the first time range, and the current moment is in a second preset time period, skipping performing blood pressure measurement on the user.

8. The method according to any one of claims 1 to 7, wherein the method further comprises:
if the heart rate of the user is greater than the first heart rate threshold, the current moment is in the first time range, and duration in which the heart rate of the user is greater than the first heart rate threshold is less than second preset duration, skipping performing blood pressure measurement on the user.

9. The method according to any one of claims 1 to 8, wherein the method further comprises:
if the heart rate of the user is greater than a third threshold and less than a fourth threshold, skipping performing blood pressure measurement on the user, wherein the third heart rate threshold is greater than or equal to the second heart rate threshold, and the fourth heart rate threshold is less than or equal to the first heart rate threshold.

10. The method according to claim 2, wherein the first signal comprises a first included angle that is between the wearable device and a horizontal plane and that is detected by the gyroscope sensor, and the first condition comprises that the first included angle is in a first preset angle range.

11. The method according to claim 4, wherein the third signal comprises a second included angle that is between the wearable device and a horizontal plane and that is detected by the gyroscope sensor.

12. The method according to claim 11, wherein the determining a second blood pressure value based on the third signal and the fourth signal comprises:
determining a third blood pressure value based on the third signal, and determining a fourth blood pressure value based on the fourth signal; and
determining the second blood pressure value based on the third blood pressure value and the fourth blood pressure value.

13. The method according to claim 1, wherein the first time range is determined in the following manner:
determined based on a work and rest habit of the user, or
determined based on time range information input by the user in a fourth user interface.

14. The method according to claim 6, wherein the second time range is determined in the following manner:
determined based on a work and rest habit of the user, or
determined based on time range information input by the user in a fourth user interface.

15. The method according to claim 6, wherein the first preset time period is determined in the following manner:
determined based on a work and rest habit of the user, or
determined based on time range information input by the user in a fourth user interface.

16. The method according to claim 7, wherein the second preset time period is determined in the following manner:
determined based on a work and rest habit of the user, or
determined based on time range information input by the user in a fourth user interface.

17. A readable medium, wherein the readable medium stores instructions, and when the instructions are executed on an electronic device, the electronic device is enabled to perform the blood pressure measurement method according to any one of claims 1 to 16.

18. An electronic device, wherein the electronic device comprises:
a memory, configured to store instructions executed by one or more processors of the electronic device; and
a processor, being one of processors of the electronic device, and configured to perform the blood pressure measurement method according to any one of claims 1 to 16.
